# EUROPEAN PATENT SPECIFICATION

(11) **EP 3 416 670 B1**
(45) Date of publication and mention of the grant of the patent: **01.06.2022**
(21) Application number: 17710483.3
(22) Date of filing: 17.02.2017
(51) Int. Cl.: A61K 38/12, A61P 31/00

(54) **A METHOD OF TREATING OR PREVENTING SEPSIS**
VERFAHREN ZUR BEHANDLUNG ODER PRÄVENTION VON SEPSIS
PROCÉDÉ DE TRAITEMENT OU DE PRÉVENTION D'UNE SEPSIE

(30) Priority: 17.02.2016 EP 16156186
(43) Date of publication of application: 26.12.2018
(73) Proprietor: Royal College of Surgeons in Ireland, Dublin 2 (IE)
(72) Inventor: KERRIGAN, Steven, Dublin 2 (IE)
(74) Representative: Purdylucey Intellectual Property
(86) International application number: PCT/EP2017/053647
(87) International publication number: WO 2017/140862

(56) References cited:
- WO-A1-2012/007137
- WO-A2-02/12501
- PRANITA P. SARANGI ET AL: "Role of [beta]1 Integrin in Tissue Homing of Neutrophils During Sepsis", SHOCK, vol. 38, no. 3, 1 August 2012 (2012-08-01) , pages 281-287, XP055289740, US ISSN: 1073-2322, DOI: 10.1097/SHK.0b013e31826136f8
- C.-C. HSU ET AL: "Improvements in endotoxemic syndromes using a disintegrin, rhodostomin, through integrin [alpha]v[beta]3-dependent pathway", JOURNAL OF THROMBOSIS AND HAEMOSTASIS, vol. 9, no. 3, 1 March 2011 (2011-03-01), pages 593-602, XP055289326, GB ISSN: 1538-7933, DOI: 10.1111/j.1538-7836.2010.04163.x
- Ralph-Heiko Mattern ET AL: "Glioma cell integrin expression and their interactions with integrin antagonists: NIH Public Access", , 25 January 2005 (2005-01-25), XP055289745, Retrieved from the Internet: URL:http://www.ncbi.nlm.nih.gov/pmc/articl es/PMC1351132/pdf/nihms-5196.pdf [retrieved on 2016-07-19] & Anonymous: "Sepsis and Septic Shock - Critical Care Medicine - Merck Manuals Professional Edition", , 21 December 2015 (2015-12-21), XP055289754, Retrieved from the Internet: URL:http://web.archive.org/web/20151221120 710/http://www.merckmanuals.com/profession al/critical-care-medicine/sepsis-and-septi c-shock/sepsis-and-septic-shock [retrieved on 2016-07-19]
- Cheng Chun-Ho ET AL: "Dynamics and functional differences between dendroaspin and rhodostomin: Insights into protein scaffolds in integrin recognition : Dynamics and Functional Differences Between Den and Rho", Protein Science, vol. 21, no. 12, 1 December 2012 (2012-12-01), pages 1872-1884, XP055800388, US ISSN: 0961-8368, DOI: 10.1002/pro.2169 Retrieved from the Internet: URL:https://www.ncbi.nlm.nih.gov/pmc/artic les/PMC3575917/pdf/pro0021-1872.pdf>

## Description

### Technical Field

The invention relates to cilengitide for use in a method for the treatment or prevention of sepsis. In particular, the invention relates to cilengitide for use in a method for preventing or inhibiting the development of sepsis, severe sepsis or septic shock in a patient with a bacterial infection of the blood.

### Background to the Invention

Currently there are no approved specific treatments for the underlying pathophysiology of sepsis and therefore the management plan focuses on reducing the infection through use of aggressive intravenous antibiotic therapy often delivered in high concentrations for long periods of time. The use of antibiotics in the treatment of sepsis is not a reliable long term solution due to the rapid global emergence of antibiotic resistant strains of bacteria. According to the most recent report on antimicrobial resistance by the World Health Organisation a 'post-antibiotic' era is imminent, suggesting that the use of antibiotics in sepsis will shortly come to an abrupt end. Upon suspicion of developing sepsis, treatment with antibiotics should begin immediately, preferably within the first six hours or earlier. Initially the patient will receive broad-spectrum antibiotics, which are effective against a variety of bacteria. These antibiotics include gentamycin, cefepime, piperacillin or meropenem. Once a blood culture comes back positive for identification of the specific type of bacteria (Gram negative or Gram positive) the following antibiotics may be administered. Gram negative: amikacin or tobramycin, Gram positive: vancomycin. All antibiotics are administered intravenously.

Animal models of sepsis have been developed in an effort to create reproducible systems for studying the disease progression, pathogenesis and preliminary testing of potential therapeutic agents. However, demonstrated benefit from a therapeutic agent in animal models of sepsis has rarely been translated into success in human clinical trials. Xigris for example has recently been withdrawn from the market due to limited efficacy. Several reasons could account for these failures but most prominent is the application of theory based on incorrect or incomplete observations in the animal models. Typically, animals (mice in particular) do not develop sepsis unless forced by delivering high levels of non-commensal bacteria. Due to the lack of applicability of animal models, researchers have focused the interaction between bacteria and human blood and endothelial cells, however significant questions about the approach to investigating these interactions have been raised.

No group to date has considered αVβ3 antagonists.

A publication by Pranita P. Sarangi, et al., (SHOCK, vol. 38, no. 2, 1 August 2012), investigated the effect of an RGD peptide on beta-1-integrin in a mouse model of endotoxemia and sepsis. This disclosure differs from the current invention at least in that the RGD peptide utilised by the authors was not an αVβ3 antagonist.

C.C. Hsu, et al., (Journal of Thrombosis and Haemostasis, vol.9, no. 3, 1 March 2011) investigated the effect of rhodostomin on LPS-induced endotoxemia in a mouse model. This publication is not concerned with methods to prevent or inhibit the early development of sepsis in an individual and there is no discussion as to the use of a αVβ3 antagonist. The αVβ3 antagonist of the current invention works by preventing the adhesion of bacteria to endothelial cells. The model utilised by the authors of Hsu *et al.,* involves isolated LPS and not bacteria and, as such, Hsu *et al.,* has not demonstrated the effects of a αVβ3 antagonist in sepsis, in particular in early sepsis, as the bacteria itself is not present. Furthermore, the model utilised by Hsu *et al.,* is not a model for early sepsis or developing sepsis which does not involve LPS overload.

It is an object of the invention to overcome at least of the above-referenced problems.

### Brief Description of the Invention

The Applicant has discovered that human vascular endothelial cells under shear conditions upregulate the expression of certain extracellular receptors, including the αVβ3 integrin receptor. In addition, the Applicant has discovered that the human pathogens *Staphylococcus aureus* and *Escherichia.coli* bind to human vascular endothelial cells under shear conditions through the interaction with the vascular endothelial αVβ3 integrin receptor. In particular, S. *Aureus* clumping factor A binds to fibrinogen which is crosslinked to αVβ3, and *E.coli* liposolysaccharide (LPS) binds directly αVβ3. The inventors have now found that in fact *Escherichia coli* outer membrane protein A binds to the endothelial cell major integrin, αVβ3. The Applicant has also discovered that blocking the receptor with a αVβ3 antagonist such as cilengitide causes a dose-dependent reduction in adhesion of both gram positive and gram negative bacteria to vascular endothelial cells. As bacterial adhesion to vascular endothelial cells is a critical event in the development of bacterial sepsis, in particular the progression of blood infection to sepsis, severe sepsis and septic shock, the invention relates to the treatment or prevention of sepsis by administration, ideally intravenous administration, of a αVβ3 antagonist to a patient. Moreover, as blocking the vascular endothelial αVβ3 integrin with a αVβ3 antagonist causes a reduction in adhesion of both gram positive and gram negative bacteria, the therapeutic intervention does not require blood cultures to be measured in advance and can be initiated as soon a blood infection is suspected or has been detected.

The invention is as outlined by the appended claims.

Accordingly, the invention relates to cilengitide for use in the treatment or prevention of sepsis in a patient, in which cilengitide is delivered to the patient intravenously.

In one embodiment, the patient has, or is suspected of having, a bacterial infection of the blood.

In one embodiment, the use of the invention is specifically for early prevention or inhibition of the development of sepsis in a patient having or suspected of having a bacterial infection of the blood.

In one embodiment, the use of the invention is specifically for early prevention or inhibition of the development of severe sepsis or septic shock in a patient having or suspected of having sepsis or an infection of the blood.

In one embodiment, the use of the invention is specifically for treatment of severe sepsis or septic shock in a patient.

In one embodiment, the use of the invention additionally comprises treatment with an intervention selected from: intravenous antibiotics; intravenous fluids; and organ-specific intervention.

In one embodiment, a dosage of less than 10mg/m² is administered to the patient. In one embodiment, a dosage of less than 1mg/m² is administered to the patient.

Described herein is a pharmaceutical composition comprising a therapeutically effective amount cilengitide and an antibiotic typically formulated for intravenous delivery. In one embodiment, the antibiotic is a broad spectrum antibiotic. Examples include gentamycin, cefepime, piperacillin or meropenem. In one embodiment, the antibiotic is a gram-negative specific antibiotic. Examples include amikacin or tobramycin. In one embodiment, the antibiotic is a gram-positive specific antibiotic. Examples include vancomycin. In one embodiment, the pharmaceutical composition comprises less than 10mg/m². In one embodiment, a dosage of less than 1mg/m² is administered to the patient.

Described herein is a method of treating or preventing sepsis in a patient, comprising intravenously administering cilengitide as described herein. In an embodiment, the method is for early prevention or inhibition of the development of sepsis in a patient having or suspected of having a bacterial infection of the blood. In an embodiment, the method is for early prevention or inhibition of the development of severe sepsis or septic shock in a patient having or suspected of having sepsis. In an embodiment, the method is for treatment of severe sepsis or septic shock in a patient.

### Brief Description of the Figures

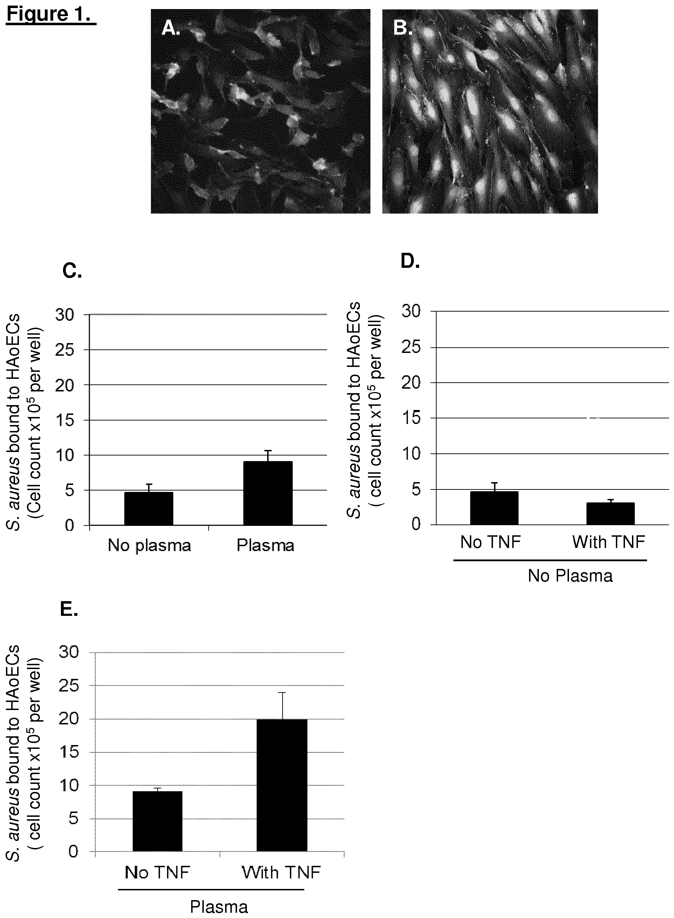

**Figure 1****.** *The effect of shear and conditions necessary for bacterial binding to Human endothelial cells.* Human endothelial cells were grown either statically **(A)** or under physiological shear conditions of 10dynes/cm² **(B).** S. aureus were allowed to adhere to the sheared human endothelial cells in the absence or presence of human plasma **(C)** and/or in the absence **(D)** or presence **(E)** of tumour necrosis factor alpha (TNFα). Once these conditions were established we investigated the ability of S. aureus to bind to human endothelial cells under static or shear conditions in the presence of plasma and TNFa **(F).** Our results indicate that S. aureus binds significantly more to sheared human endothelial cells in the presence of plasma and TNFa.

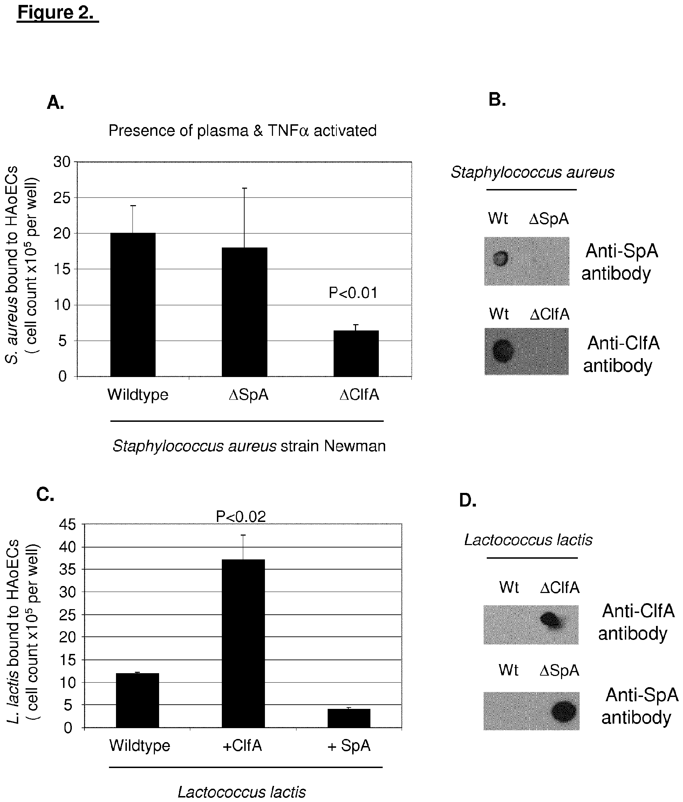

**Figure 2****.** *Identification of the major cell wall protein on S. aureus that binds to sheared human endothelial cells.* S. aureus bound to human endothelial cells in the presence of human plasma and TNFa. Using a strain deficient in expression of the protein A expressed on the bacteria surface failed to affect S. aureus binding, however using a strain deficient in expression of the major cell wall protein clumping factor A significantly reduced binding **(A).** Dot blots were used to confirm the lack of expression of protein A and Clumping factor A **(B).** L. lactis is an excellent surrogate host previously used for determining function of S. aureus proteins of interest. Over expression of protein A in surrogate host L. lactis failed to restore binding to the human endothelial cells. Over expression of clumping factor A in surrogate host L. lactis significantly increased binding to the human endothelial cells **(C).** Dots blots were used to confirm over expression of ClfA and protein A respectively **(D).** These results suggest that the major cell wall protein ClfA is critical for S. aureus binding to human endothelial cells.

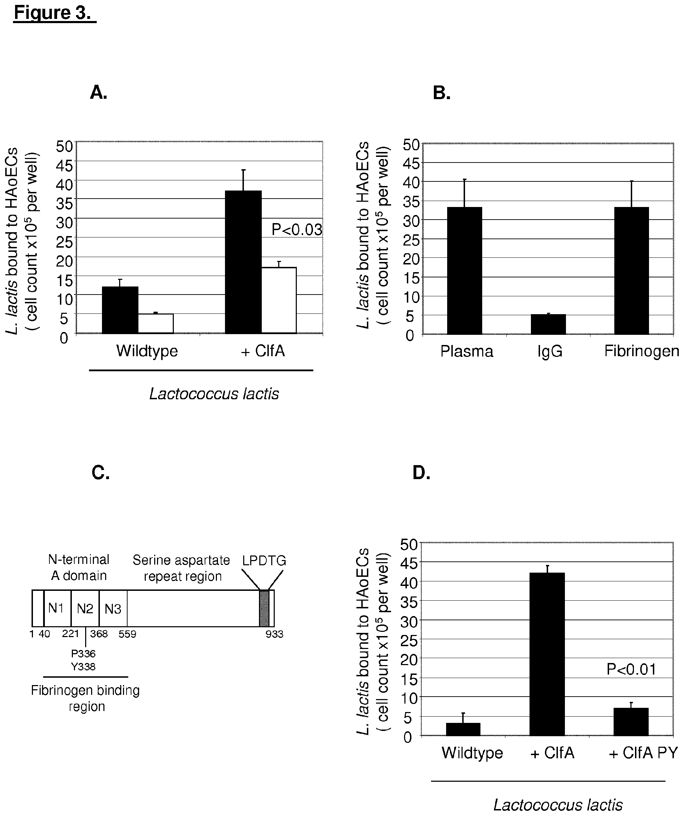

**Figure 3****.** *Identification of the role of human plasma proteins in S. aureus Clumping factor A binding to human endothelial cells.* L. lactis expressing ClfA binding to human endothelial cells was significantly reduced when ClfA plasma proteins were removed **(A).** Addition of IgG only failed to reinstate L. lactis ClfA binding to the human endothelial cells. Addition of fibrinogen only completely restored L. lactis binding to the human endothelial cells **(B).** Deletion of the amino acids in ClfA **(C)** critical for fibrinogen binding significantly reduced the interaction with the human endothelial cells **(D).** These results suggest that fibrinogen is key for ClfA to crosslink and bind to the human endothelial cells.

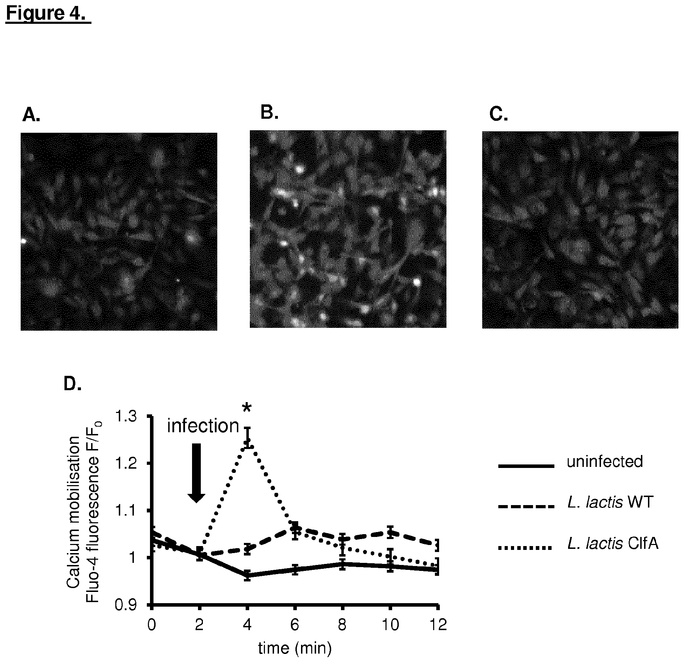

**Figure 4****.** *Intracellular calcium release in human endothelial cells as α result of ClfA*/*fibrinogen binding.* Uninfected resting human endothelial cells do not release intracellular calcium **(A and D).** However upon ClfA / fibrinogen binding to the human endothelial cells there is a spike in calcium release at around 4 minutes post infection **(B and D).** Using a strain deficient in expression of ClfA failed to trigger the intracellular signal that results in calcium release **(C and D).** These results suggest that following ClfA / fibrinogen binding to human endothelial cells an intracellular signal is generated.

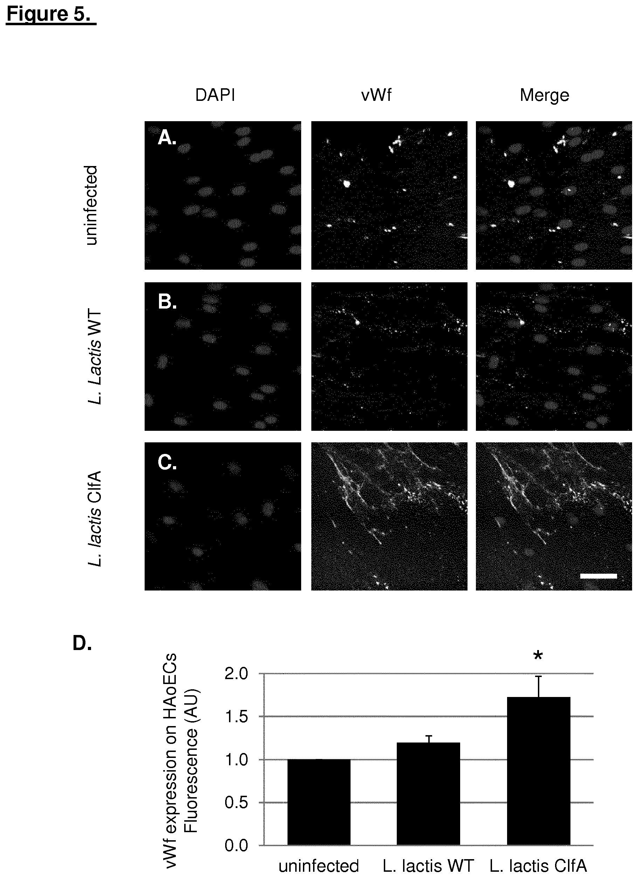

**Figure 5****.** *Mobilisation of the Weibel Palade bodies and secretion of vonWillebrand factor following ClfA*/*Fibrinogen binding.* Uninfected resting human endothelial cells **(A and D)** or L. lactis mock transfected cells **(B and D)** do not mobilise the Weibel Palade bodies or secrete vonWillebrand factor onto the surface of the endothelial cells. Over expression of ClfA in L. lactis led to mobilisation of the Weibel Palade bodies and secretion of vWf onto the surface of human endothelial cells **(C and D).**

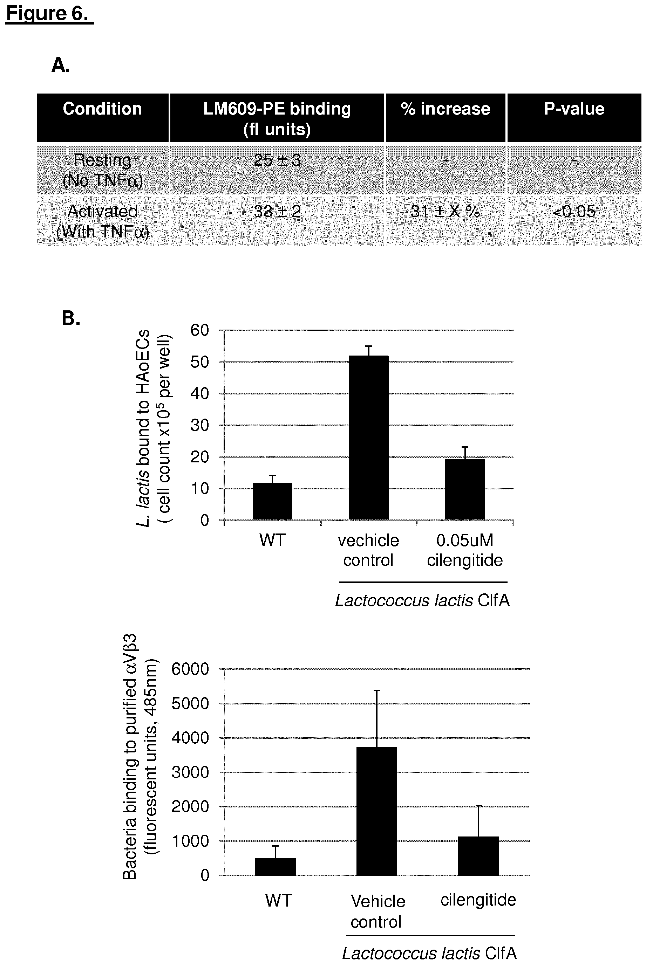

**Figure 6****.** *Identification of the endothelial cell receptor that recognises S. aureus ClfA and fibrinogen that mediated the interaction.* Upon activation with low concentration TNFa human endothelial cells αVβ3 is significantly upregulated **(A).** Binding of L. lactis expressing ClfA was significantly reduced following pretreatment of the human endothelial cells with an inhibitor of αVβ3, cilengitide **(B).** Pretreating the purified receptor aVb3 with cilengitide also significantly inhibited L. lactis ClfA from binding **(C).** These results suggest that αVβ3 is the receptor responsible for binding to fibrinogen and crosslinking to S. aureus ClfA.

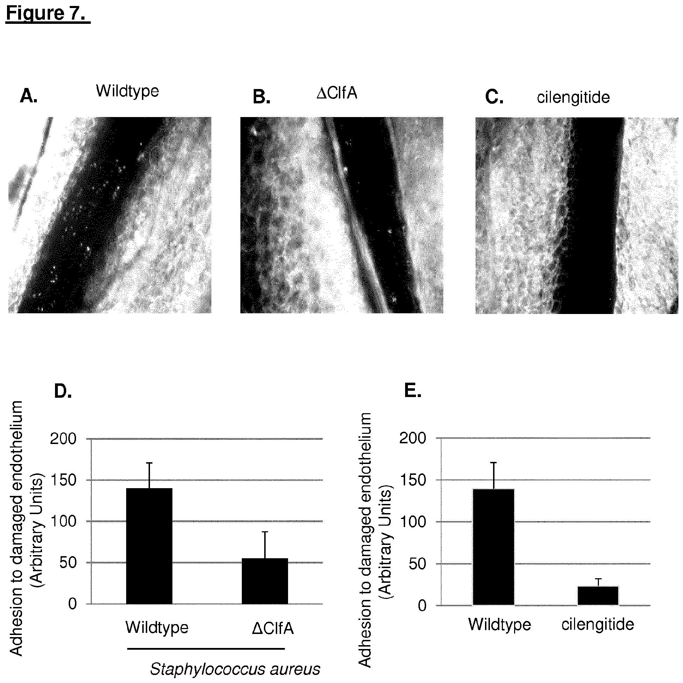

**Figure 7****.** *In vivo, venous mesenteric perfusion model to demonstrate that bacterial adhesion to endothelial cells is inhibited by cilengitide.* Wildtype S. aureus adhered to activated murine endothelial cells **(A).** Using a strain deficient in expression of ClfA significantly adhered less to the endothelial cells **(B and D).** S. aureus failed to significantly bind murine endothelial cells in the presence of cilengitide administered intravenously **(C and E).**

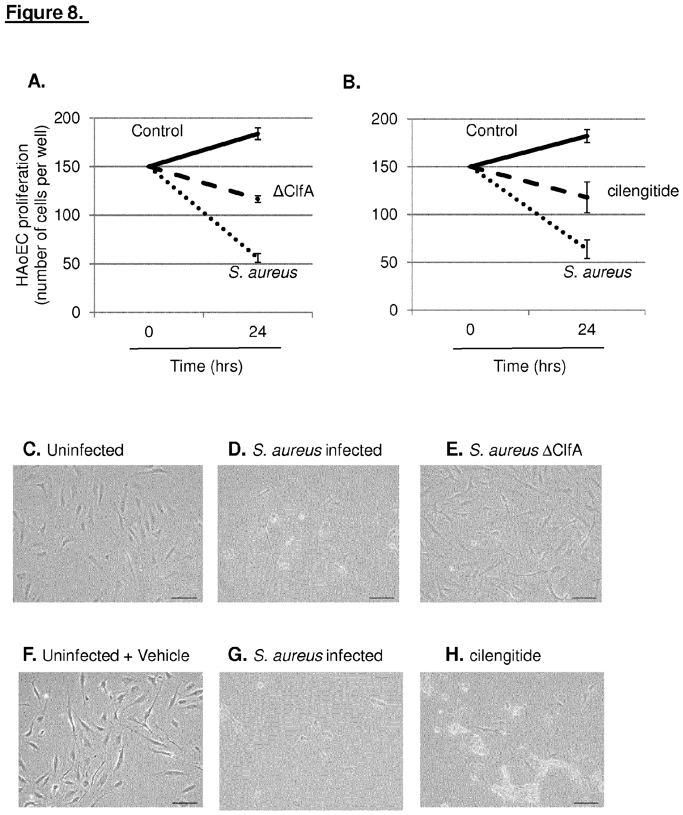

**Figure 8****.** *The effect of S. aureus on human endothelial cell proliferation.* Human endothelial cells proliferate over 24 hrs in growth media **(C and F).** Addition of S. aureus to the endothelial cells significantly reduced human endothelial cell proliferation over a 24hr period **(D and G).** Using a strain deficient in expression of ClfA significantly recovered the ability of S. aureus to inhibit human endothelial cell proliferation **(E).** Equally, addition of cilengitide to the human endothelial cells also significantly recovered the ability of S. aureus to inhibit proliferation **(H).**

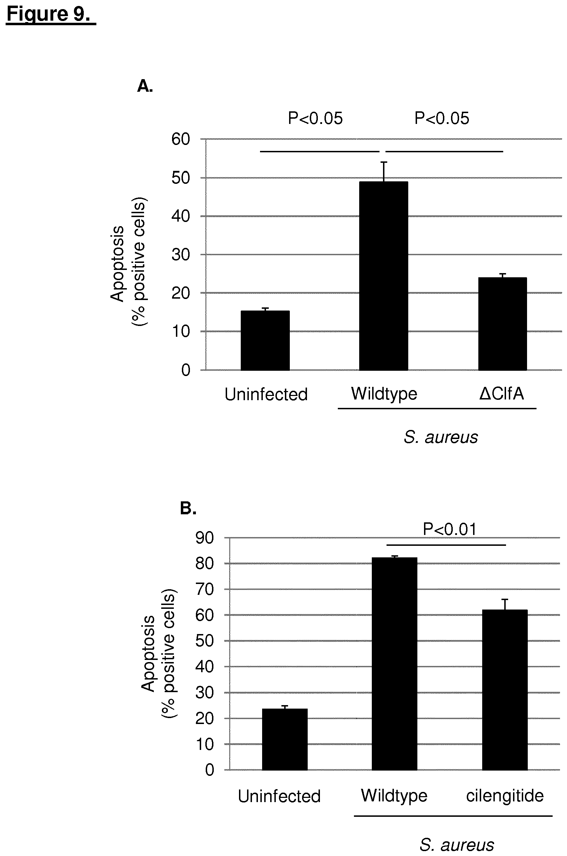

**Figure 9****.** *The effect of S. aureus on human endothelial cell apoptosis.* Based on the observation that S. aureus inhibits human endothelial cell proliferation we investigated if this is due to apoptosis. Healthy human endothelial cells have low levels of apoptosis, however addition of S. aureus significant apoptosis is observed. Using a strain deficient in expression of ClfA significantly reduced the apoptosis induced by S. aureus **(A).** Addition of cilengitide to the human endothelial cells also significantly reduced the ability of S. aureus to induce apoptosis **(B).**

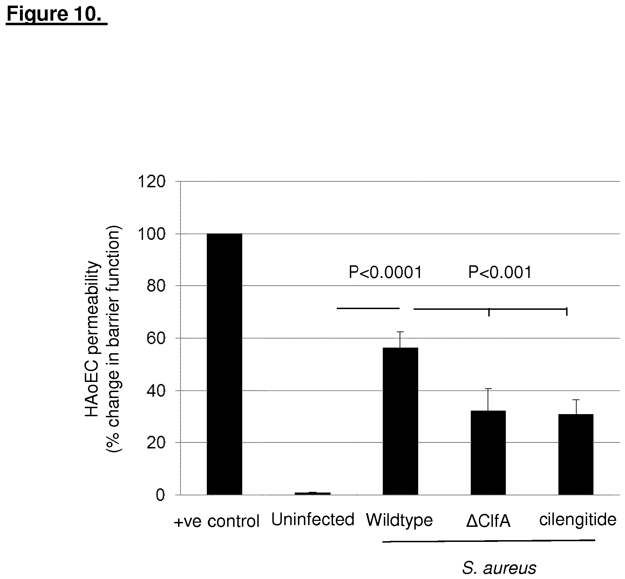

**Figure 10****.** *The effect of S. aureus on human endothelial cell permeability.* If human endothelial cells are becoming apoptotic it may lead to break down in barrier function. Uninfected human endothelial cells form tight junctions that prevents passage of fluid through the endothelial layer. Addition of S. aureus disrupts this barrier and results in significant passage of fluid across the monolayer of cells. Using a strain deficient in expression of ClfA significantly reduced this disruption in barrier permeability. Similarly, addition of cilengitide to the human endothelial cells also significantly reduced the amount of permeability of the barrier following S. aureus infection.

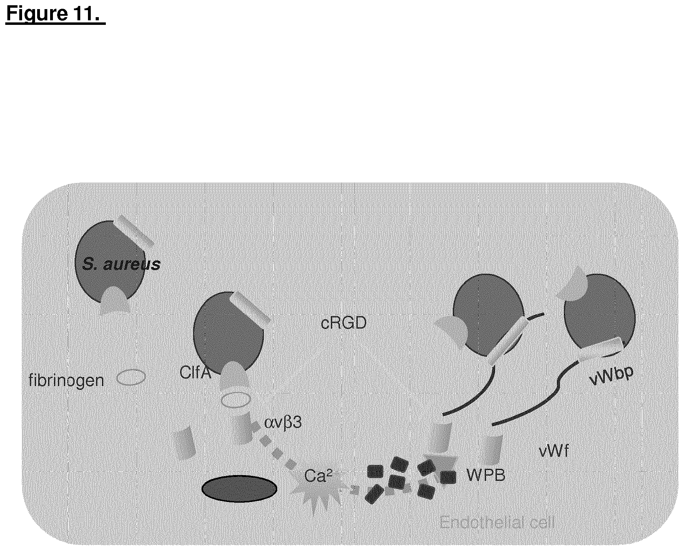

**Figure 11****.** *Model of the S. aureus interaction with human endothelial cells.* S. aureus clumping factor A binds soluble plasma fibrinogen. This in turn crosslinks the bacteria to aVb3 expressed on endothelial cells. Once bound an intracellular signal is generated which results in a significant rise in calcium and secretion of vonWillebrand factor. Additional signals result in loss of proliferation, induced apoptosis and increased vascular permeability. Together these changes cause dysregulation of normal endothelial cell function. Addition of cilengitide significantly reduces all of these dysfunctional messages.

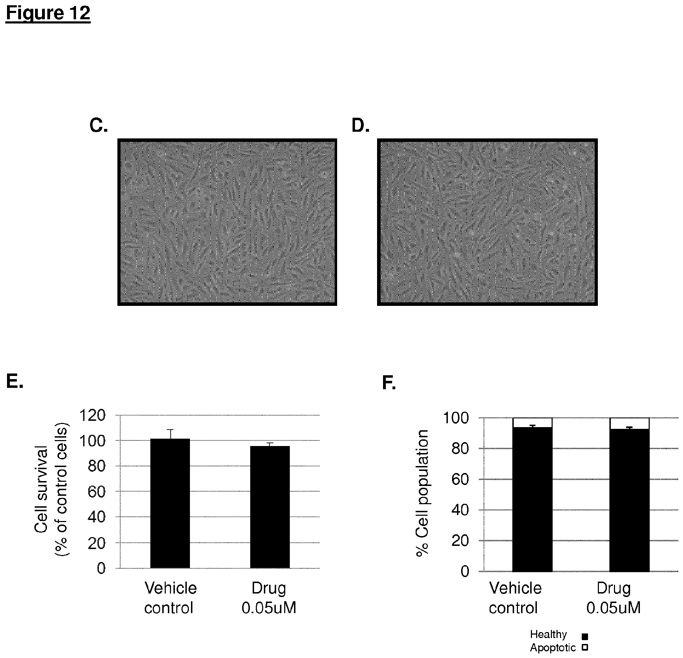

**Figure 12****.** Addition of cilengitide does not cause toxicity in human endothelial cells.

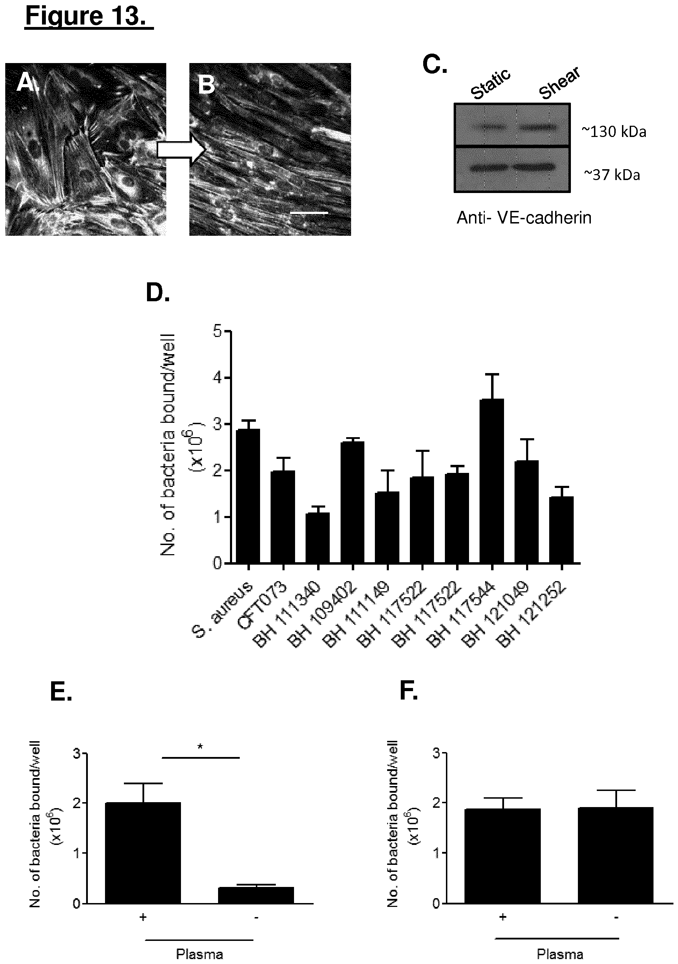

**Figure 13****.** *Escherichia coli binding to sheared Endothelial cells under static conditions.* Endothelial cells were sheared to replicate the physiological state they find themselves in the human vasculature. Endothelial cells were fixed, permeabilized and incubated with antibody against a-actin (primary) and Alexa Fluor^{®} 488-conjugated (secondary). Nuclei were stained with DAPI. Images were acquired with a fluorescence microscopy. Images are representative of three independent experiments. Endothelial cells grown under static conditions display random orientation **(A);** whereas for endothelial cells grown under shear conditions display alignment in the direction of the flow **(B).** Consistent with this it was demonstrated that VE-cadherin expression determined by western blot of protein homogenates from static and sheared endothelial cells is upregulated in sheared endothelial cells **(C).** 9 clinical strains of *E*. *coli* were tested for their ability to bind to sheared endothelial cells and all strains bound to varying degrees **(D).** *E. coli* strain CFT073 was used as the representative strain in all experiments. Consistent with previous observations *S. aureus* (control) requires plasma in order to successfully bind to the sheared endothelial cells however *E. coli* appears to bind in the absence or presence of a plasma protein. These data suggest that *E. coli* is capable of binding to sheared endothelial cells in the absence of plasma proteins this is in contrast to S. aureus binding to endothelial cells which is dependent on plasma proteins.

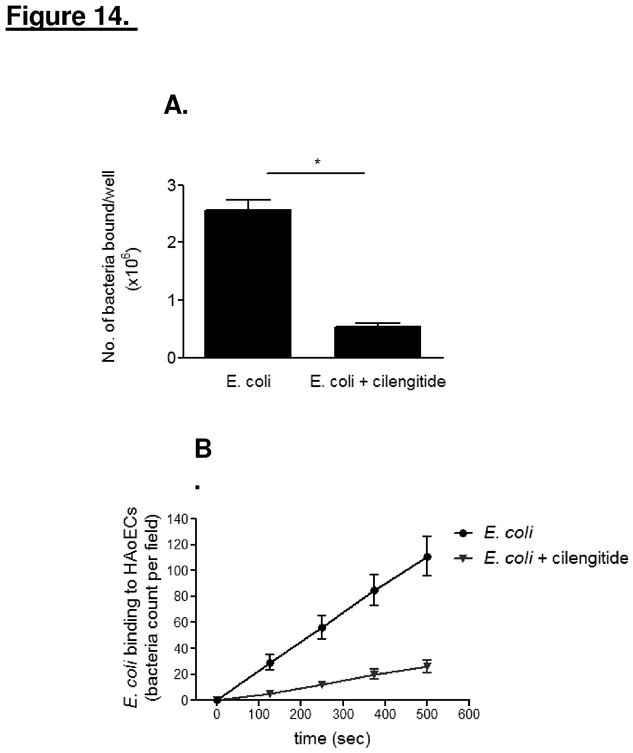

**Figure 14****.** *Escherichia coli binds to the major integrin αVβ3 expressed on endothelial cells* The effect of inhibition of α**V**β**3** integrin on binding of *E. coli* to endothelial cells was investigated. The results demonstrate that the selective α**V**β**3** integrin inhibitor, cilengitide (0.05**µ**M) significantly inhibited *E. coli* binding to endothelial cells using a static assay **(A)** or when *E. coli* are perfused over the sheared endothelial cells at 10dyns for 5 minutes **(B).** These results demonstrate that *E*. *coli* binding to endothelial cells was inhibited by cilengitide.

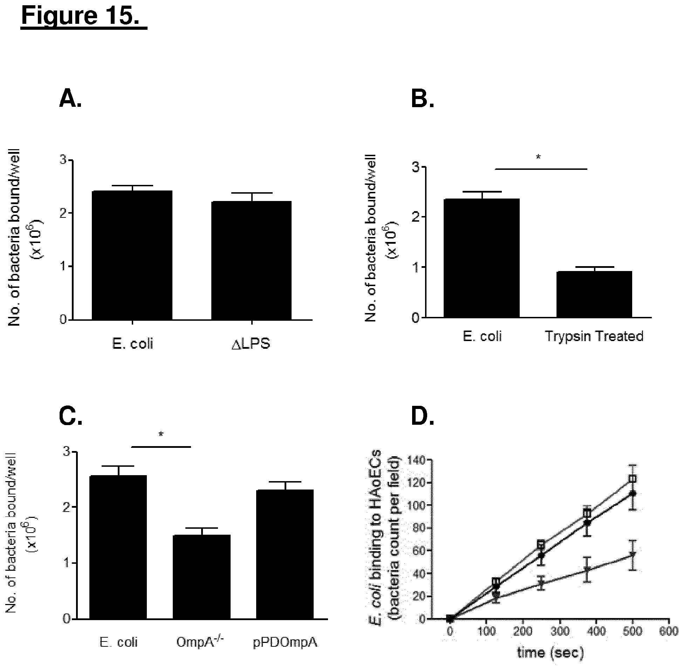

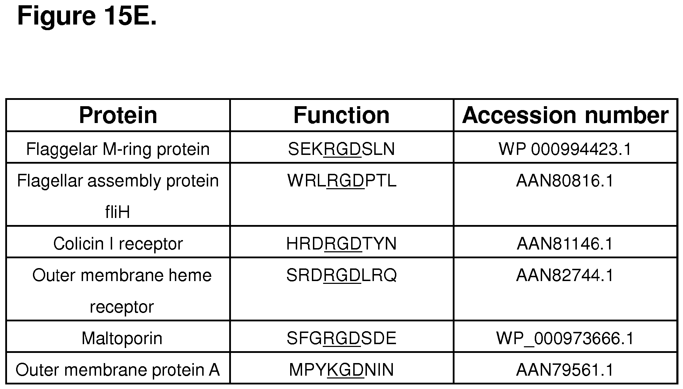

***Figure 15******.** Escherichia coli outer membrane protein A binds to the endothelial cell major integrin, αVβ3.* Using a strain deficient in LPS it was found that binding to the endothelial cells was unaffected, suggesting that LPS is not the component responsible for anchoring the bacteria to the endothelial cells **(A).** To investigate if trypsin sensitive proteins were responsible for binding to endothelial cells, the cells were pre-treated with trypsin and binding was significantly reduced **(B).** A bioinformatic approach was used to search the *E*. *coli* genome for surface proteins that contain an RGD sequence. Almost 20 proteins with an RGD or KGD sequence were identified however only 10 were expressed on the cell surface **(E).** Outer membrane protein A ompA was deleted from the parent strain CFT073 and re-introduced into the knockout to elate concerns about pleiotropic effects. The strain deficient in expression of ompA was significantly reduced in its ability to bind to the endothelial cells using both a static assay **(C)** or when the bacteria were perfused over the sheared endothelial cells **(D).** Binding was restored when the complimented strain was used, suggesting that ompA play a key role in binding to the endothelial cells **(C+D).** These results demonstrate that outer membrane protein A is ubiquitously expressed on the surface *E. coli* strains and expresses the integrin recognition motif RGD. Deletion of ompA significantly reduces the ability of E. coli from binding to the endothelial cells.

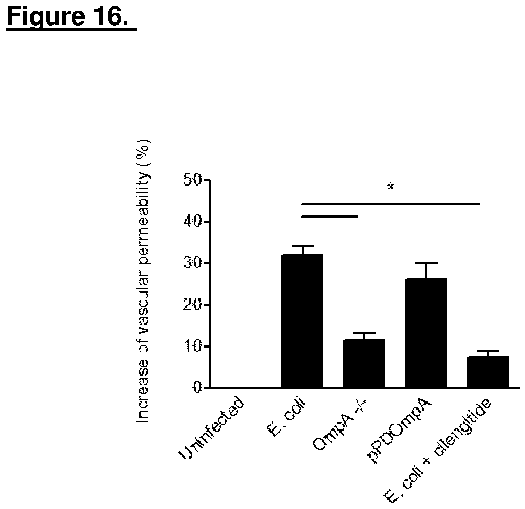

**Figure 16****.** *Escherichia coli ompA binding to αVβ3 leads to an increase in permeability in endothelial cells.* One of the characteristic signs of sepsis is increased vascular permeability. The effect of binding *of E. coli* to endothelial cells on permeability was investigated. Upon E. *coli* binding, there was a 30% increase in permeability and the strain deficient in ompA significantly reduces permeability. Addition of the complimented ompA strain resulted in an increase in permeability which is comparable to the parent *E. coli* control. Most importantly, *E. coli* induced increase in permeability was significantly reduced permeability following preincubation αVβ3 results in a signal that triggers an increase in fluid leakage and permeability. These data illustrate that *E*. *coli* binding to endothelial cells results in a significant increase in permeability (~30%). Using a strain deficient of ompA or preincubating the endothelial cells with cilengitide significantly reduces permeability.

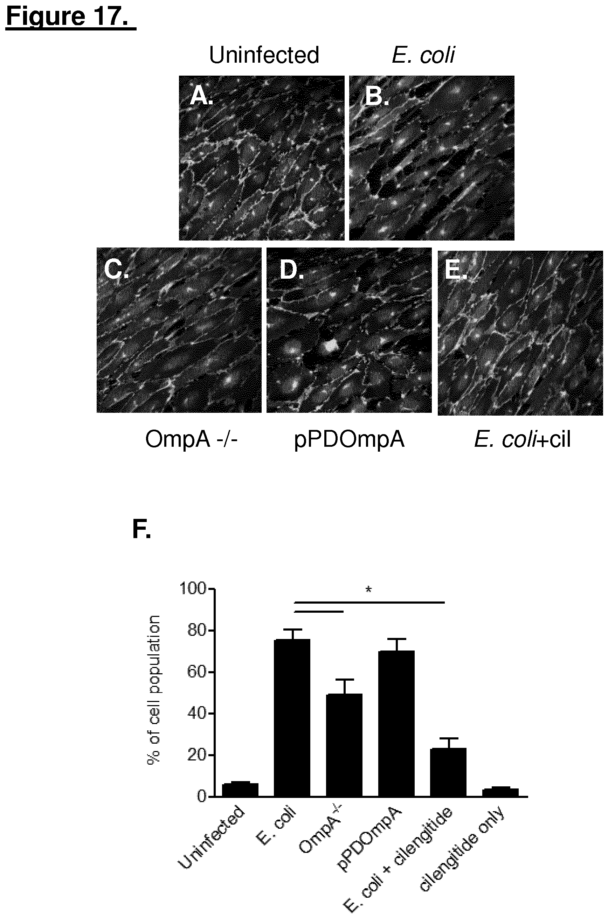

**Figure 17****.** *Escherichia coli ompA binding to* αVβ3 *results in loss of tight junction protein and increase in apoptosis.* An increase in permeability is due to a separation of the endothelial cells. To investigate, tight junction formation was assessed. Immunostaining for VE-cadherin demonstrated tight junction formation in the absence of infection **(A)** however there was a loss of connections following *E. coli* infection **(B).** Using the strain deficient in ompA recovered the VE-cadherin tight junction formation **(C).** The complimented strain also significantly led to loss of tight junction formation **(D).** Finally pre-treating the endothelial cells with cilengitide also led to recovery of tight junction formation following *E. coli* infection **(E).** It was also investigated if the loss of tight junction formation was due to apoptosis. To determine this, Annexin V exposure on the cell surface, a hall mark of apoptotic cells, was assessed by flow cytometry. Uninfected endothelial cells have a low level of apoptosis, however upon addition *of E. coli* to the endothelial cells, apoptosis was significantly increased. Addition *of E. coli* ΔompA caused a significantly lower extent of apoptosis in the endothelial cells **(F).** Addition of the complimented ompA strain also induced apoptosis comparable to the *E. coli* control. Preincubation of endothelial cells with cilengitide led to a significant reduction in *E. coli* induced apoptosis. Addition of cilengitide alone to the endothelial cells failed to affect to the viability of cells. These data demonstrate that *E*. *coli* binding to sheared endothelial cells results in separation of the endothelial cells resulting in increased permeability and that ompA binding to αVβ3 generates a signal resulting in programmed cell death (apoptosis).

### Detailed Description of the Invention

Definitions:
"αVβ3" is a member of the integrin supergene family of cell surface glycoprotein receptors that promote cellular adhesion. It binds to a variety of plasma and extracellular matrix proteins containing the conserved RGD amino acid sequence and modulates cell adhesion. It is highly expressed in osteoclasts where it plays a role in bone resorption, and is also abundant in vascular smooth muscle cells and endothelial cells, and in some tumour cells, where it is involved in angiogenesis and cell migration (Felding et al, Curr Opin Cell Biol. 1993;5;864-868). "vascular endothelial αVβ3" refers to the αVβ3 integrin expressed by vascular endothelial cells.
"αV domain" refers to the αV domain of the vasacular endothelial αVβ3 integrin The sequence of human vascular endothelial αV domain is provided in SEQUENCE ID NO: 1:
   *ITAV_HUMAN Integrin alpha-V OS=Homo sapiens (SEQUENCE ID NO: 1)*
"β3 domain" refers to the β3 domain of the vascular endothelial αVβ3 integrin. The sequence of human vascular endothelial β3 domain is provided in SEQUENCE ID NO: 2:
   *ITB3_HUMAN Integrin beta-3 OS=Homo sapiens (SEQUENCE ID NO: 2)*
"αVβ3 antagonist" refers to a molecule that can bind to a αVβ3 integrin. Examples include peptides {cyclic and noncyclic}, non-peptide ligands, disintegrins, peptidomimetics, antibodies, and gene inhibitors. An example of a cyclic peptide is cilengitide. Examples of antibody antagonists are described in the literature, for example US5652110. αVβ3 antagonists are also described in Millard et al {Theranostics 2011; 1:154=188}, Kim et al (Mol Pharm 2013 Oct 7, 3603-3611), Pfaff et al (J Biol Chem 1994; 269, 20233-20238), Healy et al (Biochemistry 1995; 34, 3948-3955), EP2298308 (University of Southern California), Koivunen et al (J Biol Chem 1993;268; 20205-20210 and 1994;124:373-380), Ruoslahti et al (Annu Rev Cell Dev Biol. 1996; 12:697-715), and Ponce et al (Faseb J. 2001; 15; 1389-1397). In one embodiment, the αVβ3 antagonist is selective for αV integrins.
"Functional αVβ3 antagonist" refers to a αVβ3 antagonist that can bind to the vascular endothelial αVβ3 integrin and cause a decrease in binding of *S. aureus* and/or *E.Coli* bacteria to vascular nendothelial αVβ3 integrin in the *in-vitro* binding assay described below. Examples include cilengitide and variants thereof described in EP0770622. It is a routine matter for a skilled person to test the αVβ3 antagonists described in the literature using the in-vitro adhesion assay described below to identify functional αVβ3 antagonists.
"Cilengitide" is a potent integrin inhibitor for the αVβ3 receptor. It is a cyclic peptapeptide (cyclo(-RGDf(*N*Me)V-) which is selective for αV integrins (Jonczyk et al, European Patent Application No: 0770622). The synthesis and activity of the molecule was published in 1999 (Dechantsreiter et al J Med Chem 1999 Aug 12; 42(16)). Cilengitide has been indicated for treatment of cancer (EP2578225, EP2338518, EP2441464).
"Functional variant of Cilengitide" means the variants of cilengitide described in US6001961 which are integrin inhibitors for the αVβ3 receptor, specifically the cyclic peptides described in Sequence ID NO's 1 to 40 (US6001961, columns 12-13).
"Low molecular weight antagonist" means an antagonist that has a molecular of less than 50 KDa. In one embodiment, the low molecular weight antagonist has a molecular weight of less than 20 KDa. In one embodiment, the low molecular weight antagonist has a molecular weight of less than 10 KDa. In one embodiment, the low molecular weight antagonist has a molecular weight of less than 5 KDa. In one embodiment, the low molecular weight antagonist has a molecular weight of less than 4 KDa. In one embodiment, the low molecular weight antagonist has a molecular weight of less than 3 KDa. In one embodiment, the low molecular weight antagonist has a molecular weight of less than 2 KDa. In one embodiment, the low molecular weight antagonist has a molecular weight of less than 1 KDa.
"αVβ3 antibody" means an antibody that binds to the αVβ3 integrin. In one embodiment, the antibody binds specifically to the αVβ3 or αVβ5 integrin. In one embodiment, the antibody binds specifically to the αVβ3 integrin. Examples of αVβ3 antibodies are described in the literature (Liu et al Drug Dev Res 2008; 69(6) 329-339) and are commercially available, for example: MAB1976, a mouse monoclonal antibody commercially available from MerckMillipore (Clone LM609), also known as AVASTIN; Vitaxin I, a humanised monoclonal antibody (Coleman et al, Circulation Research 1999; 84; 1268-1276); ABEGRIN, a derivative of Vitaxin I manufactured by Medimmune; CNTO95, a fully humanised antibody that recognises multiple αV integrins and binds to αVβ3 or αVβ5 integrins with high affinity (Trika et al. Int J Cancer 2004;110; 326-335); c7E3 Fab (ABCIXIMAB or REOPRO) and c7E3(Fab')2, mouse-human chimeric and murine mAb fragments of the parent intact murine mAb 7E3 (Trikha et al. Cancer Res 2002;62;2824-2833 and Varner et al. Angiogenesis 1999;3;53-60); 17E6 antibody (Mitjans et al J Cell Sci 1995; 108; 2825-2838; The term "αVβ3 antibody" includes antibodies that selectively bind to the αV domain, the β3 domain, or both the αV and β3 domains. The term "functional αVβ3 antibody" means an αVβ3 antibody that binds to the vascular endothelial αVβ3 integrin and is capable of at least partially blocking adhesion of *S. aureus* or *E.coli* bacteria to endothelial cells in the *in-vitro* adhesion assay described below.
"Disintegrin" means the family of low molecular weight RGD containing cysteine rich peptides derived from viper venoms that can bind to αVβ3 and block its function. Examples include Trigramin, Kistrin, Bitistatin, Barbourin, Echistatin, Contortrostatin, all described or referenced in Liu et al Drug Dev Res 2008; 69(6) 329-339.
"Peptidomimetics" means compounds containing non-peptidic structural elements that are capable of mimicking the biological actions of peptidic αVβ3 antagonists. Examples include SC-68448 and SCH221153 both described or referenced in Liu et al Drug Dev Res 2008; 69(6) 329-339, and IS201 described in Bello et al (Neurosurgery 2003:52; 177186).
"Gene inhibitors" means a nucleic acid that can cause reduced expression of αVβ3. In some embodiments, the agent is a nucleic acid. A nucleic acid agent can be selected from, for example, siRNA, shRNA, miRNA, anti-microRNA, antisense RNA or oligonucleotide, aptamer, ribozyme, and any combinations thereof. When the agent is a nucleic acid, the agent itself can be administered to the subject or a vector expressing/encoding the agent can be administered to the subject. In some embodiments, the vector expressing/encoding the agent is an Adeno-associated virus (AAV) vector.
"Infection of the blood" or "blood infection" means a microbial infection of the blood, for example a bacterial, viral or yeast blood infection. In one embodiment, the term means bacterial infection of the blood. A patient with a bacterial blood infection is often referred to as "bacteraemic". In one embodiment, the term means bacterial infection of the blood where the bacteria is a sepsis causing bacteria. In one embodiment, the term means bacterial infection of the blood with a bacterial genus selected from *Staphlyococcus, Streptococcus, Escherichia, Pseudomonas, Klebsiella.* In one embodiment, the term means bacterial infection of the blood with a bacterial species selected from *Staphylococcus aureus, Streptococcus pyogenes, Escherichia coli, Pseudomonas aeruginosa, Klebsiella spp.* Causitive agents of bacterial sepsis are more fully described in Ramachandran et al. (Virulence 2014 Jan 1; 5(1); 213-218).

Sepsis is diagnosed when a patient exhibits at least two of the following symptoms, plus a probable or confirmed infection:
- Body temperature above 101 F (38.3 C) or below 96.8 F (36 C)
- Heart rate higher than 90 beats a minute
- Respiratory rate higher than 20 breaths a minute

Severe sepsis is diagnosed if the patient also exhibit at least one of the following signs and symptoms, which indicate an organ may be failing:
- Significantly decreased urine output
- Abrupt change in mental status
- Decrease in platelet count (thrombocytopenia)
- Difficulty breathing
- Abnormal heart pumping function
- Abdominal pain

Septic shock is diagnosed if in addition to the symptoms of severe sepsis the patient also displays extremely low blood pressure that doesn't adequately respond to simple fluid replacement.

In this specification, the term "treating" refers to administering a αVβ3 antagonist cilengitide to an individual that has or is suspected of having a blood infection with the purpose to cure, heal, prevent, alleviate, relieve, alter, remedy, ameliorate, or inhibit the development of sepsis, including severe sepsis or septic shock.In a preferred embodiment, the term means preventing or inhibiting the development of sepsis in an individual with a blood infection. In one embodiment, the patient is administered a therapeutically effective amount of the αVβ3 antagonist. The term "therapeutically effective amount" refers to the amount of the αVβ3 antagonist that is required to confer the intended therapeutic effect in the individual, which amount will vary depending on the type of antagonist, route of administration, status of infection or sepsis, and possible inclusion of other therapeutics or excipients. In one embodiment, the therapeutically effective amount is less than 100mg/m². "mg/m²" means mg per m² of body surface area, and a method of calculating body surface area is provided at www.halls.md/body-surface-area/bsa.htm*.* In one embodiment, the therapeutically effective amount is less than 50mg/m². In one embodiment, the therapeutically effective amount is less than 10mg/m². In one embodiment, the therapeutically effective amount is less than 5mg/m². In one embodiment, the therapeutically effective amount is less than 1mg/m². In one embodiment, the therapeutically effective amount is 0.001 to 50mg/m². In one embodiment the therapeutically effective amount is 0.001 to 10mg/m². In one embodiment, the therapeutically effective amount is 0.001 to 1.0mg/m². In one embodiment, the therapeutically effective amount is 0.01 to 10mg/m². In one embodiment, the therapeutically effective amount is 0.1 to 10mg/m². In one embodiment, the therapeutically effective amount is 0.001 to 1.0mg/m². In one embodiment, the therapeutically effective amount is 0.01 to 1mg/m². In one embodiment, the therapeutically effective amount is 0.1 to 1mg/m². In one embodiment, the therapeutically effective amount is administered once daily for a period 2 to 14 days. In one embodiment, the methods and uses of the invention apply especially to patients having or suspected of having a bacterial infection of the blood and for the purpose of inhibiting or preventing the development of sepsis. In another embodiment, the methods and uses of the invention apply to patients that are at risk of bacterial blood infection (i.e. immunocompromised patients, trauma patients, and patients having undergone major surgery such as cardiothoracic surgery) and for the purpose of preventing or inhibiting bacterial infection of the blood. In one embodiment, the methods and uses of the invention apply especially to patients having or suspected of having sepsis and for the purpose of inhibiting or preventing the development of severe sepsis or septic shock. In one embodiment, the method and uses of the invention are for a patient in need thereof. The term "patient in need thereof' refers to a person who has or is suspected of having or developing sepsis. The term "prevention of sepsis" means preventing or inhibiting the development of sepsis in a patient with a blood infection, or preventing or inhibiting the development of sever sepsis in a patient with a sepsis, or preventing or inhibiting the development of septic shock in a patient with severe sepsis.

To practice the use of this invention, cilengitide is administered intravenously. The term "parenteral" as used herein includes subcutaneous, intracutaneous, intravenous, intramuscular, intraarticular, intraarterial, intrasynovial, intrasternal, intrathecal, intralesional, and intracranial injection or infusion techniques. A sterile injectable composition, e.g., a sterile injectable aqueous or oleaginous suspension, can be formulated according to techniques known in the art using suitable dispersing or wetting agents (such as Tween 80) and suspending agents. The sterile injectable preparation can also be a sterile injectable solution or suspension in a nontoxic parentally acceptable diluents or solvent for example, as a solution in 1,3-butanediol. Among the acceptable vehicles and solvents that can be employed are mannitol, water, Ringer's solution and isotonic sodium chloride solution. In addition, sterile, fixed oils are conventionally employed as a solvent or suspending medium (eg. Synthetic mono-or dyglycerides). Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions can also contain a long-chain alcohol diluents or dispersant, or carboxymethyl cellulose or similar dispersing agents. Other commonly used surfactants such as Tweens or Spans or other similar emulsifying agents or bioavailablity enhancers which are commonly used in the manufacture of pharmaceutically acceptable solid, liquid, or other dosage forms can also be used for the purposes of formulation.

A composition for oral administration can be any orally acceptable dosage form including, but not limited to, capsules, tablets, emulsions and aqueous suspensions, dispersions and solutions. In the case of tablets for oral use, carriers that are commonly used include lactose and corn starch. Lubricating agents, such as magnesium stearate, are also typically added. For oral administration in a capsule form, useful diluents include lactose and dried corn starch. When aqueous suspensions or emulsions are administered orally, the active ingredient can be suspended or dissolved in an oily phase combined with emulsifying or suspending agents. If desired, certain sweetening, flavouring, or colouring agents can be added. A nasal aerosol or inhalation composition can be prepared according to techniques well known in the art of pharmaceutical formulation. A fused multicyclic compound-containing composition can also be administered in the form of suppositories for rectal administration. The carrier in the pharmaceutical composition must be "acceptable" in the sense of being compatible with the active ingredient of the formulation (and preferable, capable of stabilising it) and not deleterious to the subject to be treated. For example, one or more solubilising agents, which form more soluble complexes with the fused multicyclic compounds, or more solubilising agents, can be utilised as pharmaceutical carriers for delivery of the active compounds. Examples of other carriers include colloidal silicon dioxide, magnesium stearate, sodium lauryl sulphate, and D&C Yellow #10.

"Antibody" refers to an intact αVβ3-binding immunoglobulin in any form, including but not limited to monoclonal, polyclonal, humanized or human form, or antibody fragments that bind to αVβ3. The term includes monoclonal or polyclonal αVβ3-binding fragments with the Fc (crystallizable fragment) region or FcRn binding fragment of the Fc region, referred to herein as the "Fc fragment" or "Fc domain". In one embodiment, the antibody has the Fc region removed or modified such that it cannot interact with Protein A of bacteria. Methods of making such antibodies are described in Hay et al (Immunochemistry 12(5):373-378), Parkham et al (J. Immunol 1983:131(6):2895-2902) and Baldwin et al (J. Immunol Methods 1989 121(2): 209-217). αVβ3-binding fragments may be produced by recombinant DNA techniques or by enzymatic or chemical cleavage of intact antibodies. αVβ3-binding fragments include, inter alia, Fab, Fab', F(ab')2, Fv, dAb, and complementarity determining region (CDR) fragments, single-chain antibodies (scFv), single domain antibodies, chimeric antibodies, diabodies and polypeptides that contain at least a portion of an immunoglobulin that is sufficient to confer specific antigen binding to the polypeptide. The Fc domain includes portions of two heavy chains contributing to two or three classes of the antibody. The Fc domain may be produced by recombinant DNA techniques or by enzymatic (e.g. papain cleavage) or via chemical cleavage of intact antibodies.

The antibody may be an IgG, an IgM, an IgE, an IgA or an IgD molecule. In a preferred embodiment, the antibody molecule is an IgG, and more preferably is of the IgG1, IgG2, IgG3, or IgG4 subtype. The class and subclass of antibodies may be determined by any method known in the art, for example, by using antibodies that are specific for a particular class and subclass of antibody. Antibodies specific to αVβ3 are available commercially and are described above. The class and subclass can be determined by ELISA and Western Blots, as well as other techniques. Alternatively, the class and subclass may be determined by sequencing all or a portion of the constant domains of the heavy and/or light chains of the antibodies, comparing their amino acid sequences to the known amino acid sequences of various class and subclasses of immunoglobulins, and determining the class and subclass of the antibodies.

Antibodies are a major class of biopharmaceuticals. Antibodies for therapeutic purposes are often produced from a cell line (e.g. CHO cells, the hamster line BHK21, the human PER.C6 cell line, COS, NIH 3T3, BHK, HEK, 293, L929, MEL, JEG-3, murine lymphoid cells (including NS0 and Sp2/0-Ag 14)), including hybridomas, and are usually a single clone of a specific antibody. Antibodies used for therapeutic purposes are classified as murine, chimeric, humanized or fully human antibodies and are produced by recombinant methods. A "murine antibody" is a full mouse antibody and has only limited use in humane due to its short half-life in circulation and its high immunogenicity. A "chimeric antibody" is a genetically engineered antibody, which contains both mouse and human sequences. The ratio is approximately 33% mouse contribution and 67 % human contribution. Usually the variable domains are murine and the constant region including the Fc fragment is derived from a human IgG.

A "humanized antibody" is a genetically engineered antibody, wherein the mouse content is reduced to about 5-10%. In such cases, the six CDRs of the heavy and light chains and a limited number of structural amino acids of the murine monoclonal antibody are grafted by recombinant technology to the CDR-depleted human IgG scaffold. A fully human antibody or human antibody describes antibodies, which are made in humanized mice resulting in antibodies that do not contain any mouse sequences, or made in vitro using phage libraries or ribosome display or alternatively are obtained from human donors. In certain embodiments, chimeric, humanized or primatized (CDR-grafted) antibodies, comprising portions derived from different species or fully human antibodies, are also encompassed by the present invention. The various portions of these antibodies can be joined together chemically by conventional techniques, or can be prepared as a contiguous protein using genetic engineering techniques. For example, nucleic acids encoding a chimeric or humanized chain can be expressed to produce a contiguous protein. See, e.g., Cabilly et al., U.S. Pat. No. 4,816,567; Cabilly et al., European Patent No. 0,125,023; Boss et al., U.S. Pat. No. 4,816,397; Boss et al., European Patent No. 0,120,694; Neuberger, M. S. et al., WO 86/01533; Neuberger, M. S. et al., European Patent No. 0,194,276 B1; Winter, U.S. Pat. No. 5,225,539; and Winter, European Patent No. 0,239,400 B1. See also, Newman, R. et al., BioTechnology, 10: 1455-1460 (1992), regarding primatized antibody. See, e.g., Ladner et al., U.S. Pat. No. 4,946,778; and Bird, R. E. et al., Science, 242: 423-426 (1988)), regarding single chain antibodies.

In addition, a mixture of antibodies, termed herein as an "antibody preparation", can be used according to the methods of the present invention. Such antibody preparations include polyclonal and monoclonal mixtures of antibodies. A humanized antibody may comprise portions of immunoglobulins of different origin. For example, at least one portion can be of human origin. For example, the humanized antibody can comprise portions derived from an immunoglobulin of nonhuman origin with the requisite specificity, such as a mouse, and from immunoglobulin sequences of human origin (e.g., a chimeric immunoglobulin), joined together chemically by conventional techniques (e.g., synthetic) or prepared as a contiguous polypeptide using genetic engineering techniques (e.g., DNA encoding the protein portions of the chimeric antibody can be expressed to produce a contiguous polypeptide chain). Alternatively, a humanized antibody may be created in a transgenic or humanized animal expressing the human antibody genes (see Lonberg, N. "Transgenic Approaches to Human Monoclonal Antibodies." Handbook of Experimental Pharmacology 113 (1994): 49-101). Another example of a humanized antibody of the present invention is an immunoglobulin containing one or more immunoglobulin chains comprising a CDR of nonhuman origin (e.g., one or more CDRs derived from an antibody of nonhuman origin) and a framework region derived from a light and/or heavy chain of human origin (e.g., CDR-grafted antibodies with or without framework changes). Chimeric or CDR-grafted single chain antibodies are also encompassed by the term "humanized antibody".

Methods of preparing immunoglobulins, immunizing with antigens, and polyclonal and monoclonal antibody production can be performed as described herein, or using other suitable techniques. A variety of methods have been described. See e.g., Kohler et al., Nature, 256: 495-497 (1975) and Eur. J. Immunol. 6: 511-519 (1976); Milstein et al., Nature 266: 550-552 (1977); Koprowski et al., U.S. Pat. No. 4,172,124; Harlow, E. and D. Lane, 1988, Antibodies: A Laboratory Manual, (Cold Spring Harbor Laboratory: Cold Spring Harbor, N.Y.); Current Protocols In Molecular Biology, Vol. 2 (Supplement 27, Summer '94), Ausubel, F. M. et al., Eds., (John Wiley & Sons: New York, N.Y.), Chapter 11, (1991; Rasmussen SK, Rasmussen LK, Weilguny D, Tolstrup AB. Biotechnol Lett. 2007 Feb 20.). Polyclonal antibodies which may be used in the methods of the invention are heterogeneous populations of antibody molecules derived from the sera of immunized animals (using αVβ3-specific peptides or recombinant proteins as an immunogen). For preparation of Monoclonal antibodies directed to αVβ3, a hybridoma can be produced by fusing a suitable immortal cell line (e.g., a myeloma cell line such as SP2/0) with antibody producing cells. The antibody producing cell, preferably those of the spleen or lymph nodes, are obtained from animals immunized with the antigen of interest (αVβ3-specific peptide or recombinant protein). Hybridomas can be isolated using selective culture conditions, and cloned by limiting dilution. Cells that produce antibodies with the desired specificity can be selected by a suitable assay (e.g., ELISA). Antibodies can be purified from the plasma of human donors. This is the current approach for IVIg where immunoglobulins are isolated from normal plasma pooled from a large number of donors (see http://www.fda.gov/cber/gdlns/igivimmuno.htm and Haeney Clin Exp Immunol. 1994 July; 97(Suppl 1): 11-15.) Other suitable methods for producing or isolating antibodies of the requisite specificity can be used, including, for example, methods which select recombinant antibodies from a library, or which rely upon immunization of transgenic animals (e.g., mice) capable of producing a full repertoire of human antibodies. See e.g., Jakobovits et al., Proc. Natl. Acad. Sci. USA, 90: 2551-2555 (1993); Jakobovits et al., Nature, 362: 255-258 (1993); Lonberg et al., U.S. Pat. No. 5,545,806; Surani et al., U.S. Pat. No. 5,545,807.

An antibody may have one or more binding sites. If there is more than one binding site, the binding sites may be identical to one another or may be different. For instance, a naturally occurring immunoglobulin has two identical binding sites, a single-chain antibody or Fab fragment has one binding site, while a "bispecific" or "bifunctional" antibody has two different binding sites. An individual antibody's ability to internalize, deliver a payload, and kill a target cell can vary greatly depending on the affinity and the particular target epitope that is engaged by the antibody. For a given target in this case IL1RAPL-1 a panel of antibodies should be developed (will cover a range of binding affinities and epitopes) for ADC conjugation. The term "human antibody" includes all antibodies that have one or more variable and constant regions derived from human immunoglobulin sequences. In one embodiment, all of the variable and constant domains are derived from human immunoglobulin sequences (a fully human antibody). The term "chimeric antibody" refers to an antibody that contains one or more regions from one antibody and one or more regions from one or more other different antibodies. In one embodiment, one or more of the CDRs are derived from a specific human antibody. In a more preferred embodiment, all of the CDRs are derived from a human antibody. In another preferred embodiment, the CDRs from more than one human antibody are mixed and matched in a chimeric antibody. For instance, a chimeric antibody may comprise a CDR1 from the light chain of a first human antibody combined with CDR2 and CDR3 from the light chain of a second human antibody, and the CDRs from the heavy chain may be derived from a third antibody. Further, the framework regions may be derived from one of the same antibodies, from one or more different antibodies, such as a human antibody, or from a humanized antibody. (chimeric is not listed on page 4 -see antibody and fragments specific for the target protein).

### Experimental

### Cell culture Conditions

Primary derived Human Aortic Endothelial Cells were purchased from Promocell GmbH (Heidelberg Germany). The cells were routinely grown in Cell+ T75 tissue culture flasks containing Promocell Endothelial Cell Growth Media MV supplemented with 5% foetal calf serum, 0.4% endothelial cell growth supplement/bovine hypothalamic extract, heparin (90 mg/mL), hydrocortisone (1 mg/mL), epidermal growth factor (10 ng/mL), and antibiotics (100 U/mL penicillin, 100 mg/mL streptomycin) Cells were grown in a humidified atmosphere of 5% CO2/95% air at 37°C. For all experiments cells between passages 6 and 12 were used. HAoECs were seeded at 0.4×10⁶ into 6 well plates and grown to confluency. The HAoECs were sheared by applying 10dynes/cm2 of shear stress for 24hrs. After shearing cells were either experimented upon in the 6 well plate or harvested for seeding into adhesion assays, proliferation and permeability assays.

### Bacterial culture conditions

*Bacterial cultures of S. aureus* and *E.coli* were grown to stationary phase at 37°C in brain heart infusion broth. *L. lactis* was grown to stationary phase at 37°C in M17 Agar supplemented with glucose to a final concentration of 0.5%. Mutant strains *L. lactis* +ClfA was grown in the presence of erythromycin to a final concentration of 5ug/ml and *L. lactis* +ClfA PY was grown in the presence of chloramphenicol to a final concentration of 10ug/ml. Bacteria were harvested and washed by centrifuging at 2600 × g for 7 minutes and re-suspending in PBS pH 7.4 and adjusted to OD1 at 600nm. For experiments utilising fluorescent staining Tris Buffered Saline (TBS) pH 7.4 (50 mM Tris, 150mM NaCl) was used in place of PBS to avoid any incompatibility with phosphate groups in the buffer.

### Immunohistochemistry

Glass cover slips, (type 1, 24 × 24 mm 0.17mm thick borosilicate glass) were attached to the base of a 6 well tissue culture sealing the lower edges with a PAP pen. HAoECs were seeded into the well at 600,000 cells per well incubating for 24 hrs to assure attachment to the glass cover slips. The media in the well was replaced and the cells exposed to a shear force of 10 dyn/cm² for 24 hrs. After shearing the media was removed, the cover slip removed from the well and the cells washed with 37°C PBS. The cells were fixed with 3.7% formaldehyde in PBS for 20 minutes on ice, washed and permeabilised with 0.2% triton 100X in PBS for 5 minutes at room temperature. Liquid was removed and the cells were washed with 2 ml of PBS three times. Cells were blocked with 1.5 ml per well 5% BSA in PBS for 30 minutes at room temperature and washed three times with PBS. Monoclonal anti ZO-1 antibody was added to the cells at a 1:100 dilution in 400 µl of 5% BSA and incubated overnight at 4°C. After the cells were washed and secondary antibody Alexaflour 488, Rabbit anti goat was added at a concentration of 1:1000 in 1 ml of BSA incubating on the cells for 1 hr at room temperature in the dark. After secondary antibody staining the cells were washed three times in 1 ml of PBS. One drop of ProLong^{®} Diamond Antifade Mountant with DAPI was added to a glass microscope slide and the cover slip with cells was mounted to it. Images were acquired using an inverted fluorescence microscope (Zeiss AxioObserverZ1) equipped with plan-apochromat 63x/1.40 oil immersion objective (Ex/Em 488nm/>505nm for ZO-1 and 350/> 400nm for DAPI). Static cells for comparison to sheared were prepared in the same manner but omitting the shearing step overnight.

### Binding Assay

Sheared HAoECs were seeded into wells of a 96 well tissue culture plate at a density of 2.5×10⁴ cells per well in a volume of 100µl. Experiments were carried out either in the presence or absence of TNFα at 10 ng/ml. The HAoECs were incubated at 37°C 5% CO2 for 4 hours to facilitate attachment and cell-cell contact on the plate. Following the 4 hr incubation the media was removed and 100µl of human platelet poor plasma (PPP), 4 mg/ml fibrinogen or 1mg/ml IgG was added to each well and incubated for 1hr at 37°C 5% CO2. The wells were washed with PBS and the wells were blocked with 100 µl of 1% pre well for 1 hr at 37°C. Finally the wells were washed with 100 µl of TBS and 100 µl of SYBR green II stained bacteria was added per well at a MOI of 400. The cells were incubated for a further 1 hr at 37°C 5% CO2 and then read on a fluorescent plate reader (1420 Victor V3, Perkin Elmer, Dublin, Ireland) at 485/535 nm. The bacteria were then removed gently by pipette and the plate washed with 100 µl TBS/well. The plate was then read again at 485/535 nm. Adhered bacteria were calculated as a percent remaining from the initial reading multiplied by the number of bacteria added. Depletion of plasma was carried out by re suspending a pellet of 1 ml of OD1 *L. lactis* +ClfA in 1 ml of human hirudinised plasma. This was left on a rotator at 37°C for 1 hr, centrifuged at 3200 × g for 5 minutes and the supernatant removed. The plasma now deficient in the plasma proteins bound to by ClfA was used in the bacterial adhesion assay. Blocking of endothelial cells with c(RGDfV) was carried out by an additional step after the 4 hour incubation with TNFα and before the addition of fibrinogen. Removing the TNFα containing media 100µl of new media containing 0.05µM C(RGDfV) was added to the endothelial cells and incubated for 1 hour at 37°C. This experiment was repeated using *E. coli.*

### Dot blot

Overnight growths of wild type *S. aureus* Newman Wild, mutants, L. lactis mock transfected and mutant strains adjusted to OD1 at 600 nm in PBS and lysed ysing RIPA buffer. A spot of the lysate was placed on a nitrocellulose membrane allowed to dry at room temperature. The membrane was blocked with 5 % w/v skimmed milk proteins. Membranes were probes with anti ClfA or anti SpA antibodies followed by HRP conjugated secondary antibodies. Secondary antibody was detected by the addition of enhanced chemiluminescence solution to the membrane for 5 minutes before transferring the membrane to a radiographic cassette and exposing x-ray film to the membranes in a dark room.

### Flow Cytometry

Sheared HAoECs in a 6 well plate were exposed to 1 ng of TNFα per 25,000 endothelial cells or fresh media and were incubated for 4 hrs at 37°C. The TNFα containing media was removed and 1 ml of 4mg/ml Fg in media at 37°C for 1 hr. Cells were detached by trypsinization and pelleted. The cell pellet was washed in ice cold (4°C) PBS before being probed with anti αVβ3 antibody LM609 for 15 minutes, washed and probed with a 535 anti-mouse secondary. Cells were analysed on a flow cytometer (BD FACSCanto^{™} II Flow Cytometer).

### Proliferation assay

Sheared HAoECs were seeded into a six well plate at 150,000 cells per well in 1.6ml media containing 10 ng/ml of TNFα. The cells were incubated for 4 hrs at 37°C in a 5% CO2. The media was replaced by 1.6ml of either fresh media or media containing 0.05 µM cilengitide and incubated for 1hr. Following this media was removed and 1 ml of 4 mg/ml fibrinogen was added per well incubating for a further hour. The media was removed and the well washed with antibiotic free media. To each well 1.6ml of antibiotic free media was added containing S. aureus Newman or *S. aureus* ΔClfA for a MOI of 400. The cells were incubated at 37°C in for 24 hrs. Following incubation the media was removed from the cells and they were washed 4 times with warm PBS. The cells were detached from the 6 well plate by trypsinization counted on a haemocytometer.

### Apoptosis assay

Sheared HAoECs in a 6 well plate were exposed to 1 ng of TNFa per 25,000 endothelial cells and were incubated for 4 hrs at 37°C. The TNFα containing media was removed and 1 ml of 4mg/ml Fg in media at 37°C for 1 hr. Media was removed and replaced with 1 ml of antibiotic free media containing *S. aureus* Newman WT or Newman ΔClfA to a MOI of 400 in a final volume per well of 2 ml. The cells were incubated for 24 hrs at 37°C. Following incubation the media was removed and the wells washed 3 times with warm PBS. Cells were detached by trypsinization and pelleted. The cell pellet was washed in ice cold (4°C) PBS before being stained with a TACS^{™} Annexin V Apoptosis Kit. The cells were analysed on a flow cytometer (BD FACSCanto^{™} II Flow Cytometer).The experiment was repeated to test αVβ3 blocking by cilengitide at 0.05 µM for 1 hr between the TNFα exposure and the addition of 4 mg/ml fibrinogen for 1 hr. This experiment was repeated using *E. coli* and *E. coli* ΔompA instead of *S. aureus* Newman WT or Newman ΔClfA.

### Permeability

Endothelial barrier permeability was assessed using a transwell apparatus with the slight modifications. Sheared HAoECs were trypsinized and seeded into hanging inserts, were placed in a 6 well plate. To the lower (abluminal) chamber of the plate 4 ml of cell media was added and 2 ml of media containing 200,000 cells/ml was added to the top (luminal) chamber. The wells plates were incubated at 37°C in a 5% CO₂ and humidified atmosphere overnight to form a confluent monolayer. Media in the abluminal chamber was changed to antibiotic free media while simultaneously the media in the luminal chamber was removed and washed with antibiotic free media. Cells were infected with *S. aureus* Newman WT and *S. aureus* ΔClfA in 2 ml of antibiotic free media to infect the cells at a MOI of 400 for 3 hrs at 37°C. FITC dextran (40 kDa) was added to the top chamber for a final concentration of 250 µg/ml in 2 ml. Media samples from the abluminal chamber were taken 24hrs after infection measured on a plate reader at fluorescence Ex/Em 485/535 nm. The experiment was repeated to test αVβ3 blocking by cilengitide at 0.05 µM for 1 hr between the TNFα exposure and the addition of 4 mg/ml fibrinogen in place of plasma for 1 hr. This experiment was repeated using *E. coli* and *E. coli* ΔompA instead of *S. aureus* Newman WT or Newman ΔClfA.

### MTT cytotoxicity assay

Cell metabolic activity was measured using a MTT ((3-(4,5-dimethylthiazol-2-yl)-2,5-diphenyltetrazolium bromide) assay kit. Sheared HAoECs were seeded into a clear 96 well microtitre plate at a density of 2.5×10⁴ cells/well in 100µl in media containing 10 ng/ml TNFα. The cells were incubated for 4 hrs at 37°C. Media was removed from the wells after 4 hrs and replaced with 100 µl of fresh cell media, cell media containing a vehicle or cell media containing 0.05 µM cilengitide. The plate was incubated for a further one hour before the media was changed for 100 µl of fresh cell media with 10 µl of 12 mM MTT stock and the plate incubated at 37°C for 4 hrs. All but 25 µl of liquid was removed from each well and 10 µl of DMSO was added per well. The plate was incubated for 10 minutes at 37°C before being read on a plate reader for absorbance at 570 nm.

## Claims

1. Cilengitide for use in the treatment or prevention of sepsis in a patient, in which cilengitide is delivered to the patient intravenously.

2. Cilengitide for use according to Claim 1, for early prevention or inhibition of the development of sepsis in a patient having or suspected of having a bacterial infection of the blood.

3. Cilengitide for use according to Claim 1, for early prevention or inhibition of the development of severe sepsis or septic shock in a patient having or suspected of having sepsis.

4. Cilengitide for use according to any preceding Claim, for treatment of severe sepsis or septic shock in a patient.

5. Cilengitide for use according to any preceding Claim in which the patient is also treated with an intervention selected from: intravenous antibiotics; intravenous fluids; and organ-specific intervention.

## Patentansprüche

1. Cilengitid für die Verwendung bei der Behandlung oder Prävention von Sepsis bei einem Patienten, in der Cilengitid intravenös an den Patienten abgegeben wird.

2. Cilengitid für die Verwendung nach Anspruch 1 für eine frühzeitige Prävention oder Hemmung der Entwicklung von Sepsis bei einem Patienten, der eine bakterielle Infektion des Bluts hat oder bei dem der Verdacht dieser besteht.

3. Cilengitid für die Verwendung nach Anspruch 1 für eine frühzeitige Prävention oder Hemmung der Entwicklung von schwerer Sepsis oder septischem Schock bei einem Patienten, der Sepsis hat oder bei dem der Verdacht dieser besteht.

4. Cilengitid für die Verwendung nach einem vorstehenden Anspruch für die Behandlung von schwerer Sepsis oder septischem Schock bei einem Patienten.

5. Cilengitid für die Verwendung nach einem vorstehenden Anspruch, in der der Patient auch mit einer Maßnahme behandelt wird, die aus intravenösen Antibiotika, intravenösen Flüssigkeiten und organspezifischer Maßnahme ausgewählt ist.

## Revendications

1. Cilengitide pour une utilisation destinée au traitement ou à la prévention d'une sepsie chez un patient, le cilengitide étant administré au patient par voie intraveineuse.

2. Cilengitide pour une utilisation selon la revendication 1, destinée à la prévention précoce ou l'inhibition du développement d'une sepsie chez un patient souffrant ou suspecté comme souffrant d'une infection bactérienne du sang.

3. Cilengitide pour une utilisation selon la revendication 1, destinée à la prévention précoce ou l'inhibition du développement d'une sepsie grave ou d'un choc septique chez un patient souffrant ou suspecté comme souffrant d'une sepsie.

4. Cilengitide pour une utilisation selon l'une quelconque des revendications précédentes, destinée au traitement d'une sepsie grave ou d'un choc septique chez un patient.

5. Cilengitide pour une utilisation selon l'une quelconque des revendications précédentes, le patient étant également traité par une intervention choisie parmi : des antibiotiques par voie intraveineuse, des fluides par voie intraveineuse, et une intervention spécifique d'un organe.


### SEQUENCE LISTING

<110> ROYAL COLLEGE OF SURGEONS IN IRELAND
<120> A method of treating or preventing sepsis
<130> P11915PC00
<160> 2
<170> PatentIn version 3.5
<210> 1
   <211> 1048
   <212> PRT
   <213> Homo sapiens
<400> 1
   Met Ala Phe Pro Pro Arg Arg Arg Leu Arg Leu Gly Pro Arg Gly Leu
   1               5                   10                  15
   Pro Leu Leu Leu Ser Gly Leu Leu Leu Pro Leu Cys Arg Ala Phe Asn
               20                  25                  30
   Leu Asp Val Asp Ser Pro Ala Glu Tyr Ser Gly Pro Glu Gly Ser Tyr
           35                  40                  45
   Phe Gly Phe Ala Val Asp Phe Phe Val Pro Ser Ala Ser Ser Arg Met
       50                  55                  60
   Phe Leu Leu Val Gly Ala Pro Lys Ala Asn Thr Thr Gln Pro Gly Ile
   65                  70                  75                  80
   Val Glu Gly Gly Gln Val Leu Lys Cys Asp Trp Ser Ser Thr Arg Arg
                   85                  90                  95
   Cys Gln Pro Ile Glu Phe Asp Ala Thr Gly Asn Arg Asp Tyr Ala Lys
               100                 105                 110
   Asp Asp Pro Leu Glu Phe Lys Ser His Gln Trp Phe Gly Ala Ser Val
           115                 120                 125
   Arg Ser Lys Gln Asp Lys Ile Leu Ala Cys Ala Pro Leu Tyr His Trp
       130                 135                 140
   Arg Thr Glu Met Lys Gln Glu Arg Glu Pro Val Gly Thr Cys Phe Leu
   145                 150                 155                 160
   Gln Asp Gly Thr Lys Thr Val Glu Tyr Ala Pro Cys Arg Ser Gln Asp
                   165                 170                 175
   Ile Asp Ala Asp Gly Gln Gly Phe Cys Gln Gly Gly Phe Ser Ile Asp
               180                 185                 190
   Phe Thr Lys Ala Asp Arg Val Leu Leu Gly Gly Pro Gly Ser Phe Tyr
           195                 200                 205
   Trp Gln Gly Gln Leu Ile Ser Asp Gln Val Ala Glu Ile Val Ser Lys
       210                 215                 220
   Tyr Asp Pro Asn Val Tyr Ser Ile Lys Tyr Asn Asn Gln Leu Ala Thr
   225                 230                 235                 240
   Arg Thr Ala Gln Ala Ile Phe Asp Asp Ser Tyr Leu Gly Tyr Ser Val
                   245                 250                 255
   Ala Val Gly Asp Phe Asn Gly Asp Gly Ile Asp Asp Phe Val Ser Gly
               260                 265                 270
   Val Pro Arg Ala Ala Arg Thr Leu Gly Met Val Tyr Ile Tyr Asp Gly
           275                 280                 285
   Lys Asn Met Ser Ser Leu Tyr Asn Phe Thr Gly Glu Gln Met Ala Ala
       290                 295                 300
   Tyr Phe Gly Phe Ser Val Ala Ala Thr Asp Ile Asn Gly Asp Asp Tyr
   305                 310                 315                 320
   Ala Asp Val Phe Ile Gly Ala Pro Leu Phe Met Asp Arg Gly Ser Asp
                   325                 330                 335
   Gly Lys Leu Gln Glu Val Gly Gln Val Ser Val Ser Leu Gln Arg Ala
               340                 345                 350
   Ser Gly Asp Phe Gln Thr Thr Lys Leu Asn Gly Phe Glu Val Phe Ala
           355                 360                 365
   Arg Phe Gly Ser Ala Ile Ala Pro Leu Gly Asp Leu Asp Gln Asp Gly
       370                 375                 380
   Phe Asn Asp Ile Ala Ile Ala Ala Pro Tyr Gly Gly Glu Asp Lys Lys
   385                 390                 395                 400
   Gly Ile Val Tyr Ile Phe Asn Gly Arg Ser Thr Gly Leu Asn Ala Val
                   405                 410                 415
   Pro Ser Gln Ile Leu Glu Gly Gln Trp Ala Ala Arg Ser Met Pro Pro
               420                 425                 430
   Ser Phe Gly Tyr Ser Met Lys Gly Ala Thr Asp Ile Asp Lys Asn Gly
           435                 440                 445
   Tyr Pro Asp Leu Ile Val Gly Ala Phe Gly Val Asp Arg Ala Ile Leu
       450                 455                 460
   Tyr Arg Ala Arg Pro Val Ile Thr Val Asn Ala Gly Leu Glu Val Tyr
   465                 470                 475                 480
   Pro Ser Ile Leu Asn Gln Asp Asn Lys Thr Cys Ser Leu Pro Gly Thr
                   485                 490                 495
   Ala Leu Lys Val Ser Cys Phe Asn Val Arg Phe Cys Leu Lys Ala Asp
               500                 505                 510
   Gly Lys Gly Val Leu Pro Arg Lys Leu Asn Phe Gln Val Glu Leu Leu
           515                 520                 525
   Leu Asp Lys Leu Lys Gln Lys Gly Ala Ile Arg Arg Ala Leu Phe Leu
       530                 535                 540
   Tyr Ser Arg Ser Pro Ser His Ser Lys Asn Met Thr Ile Ser Arg Gly
   545                 550                 555                 560
   Gly Leu Met Gln Cys Glu Glu Leu Ile Ala Tyr Leu Arg Asp Glu Ser
                   565                 570                 575
   Glu Phe Arg Asp Lys Leu Thr Pro Ile Thr Ile Phe Met Glu Tyr Arg
               580                 585                 590
   Leu Asp Tyr Arg Thr Ala Ala Asp Thr Thr Gly Leu Gln Pro Ile Leu
           595                 600                 605
   Asn Gln Phe Thr Pro Ala Asn Ile Ser Arg Gln Ala His Ile Leu Leu
       610                 615                 620
   Asp Cys Gly Glu Asp Asn Val Cys Lys Pro Lys Leu Glu Val Ser Val
   625                 630                 635                 640
   Asp Ser Asp Gln Lys Lys Ile Tyr Ile Gly Asp Asp Asn Pro Leu Thr
                   645                 650                 655
   Leu Ile Val Lys Ala Gln Asn Gln Gly Glu Gly Ala Tyr Glu Ala Glu
               660                 665                 670
   Leu Ile Val Ser Ile Pro Leu Gln Ala Asp Phe Ile Gly Val Val Arg
           675                 680                 685
   Asn Asn Glu Ala Leu Ala Arg Leu Ser Cys Ala Phe Lys Thr Glu Asn
       690                 695                 700
   Gln Thr Arg Gln Val Val Cys Asp Leu Gly Asn Pro Met Lys Ala Gly
   705                 710                 715                 720
   Thr Gln Leu Leu Ala Gly Leu Arg Phe Ser Val His Gln Gln Ser Glu
                   725                 730                 735
   Met Asp Thr Ser Val Lys Phe Asp Leu Gln Ile Gln Ser Ser Asn Leu
               740                 745                 750
   Phe Asp Lys Val Ser Pro Val Val Ser His Lys Val Asp Leu Ala Val
           755                 760                 765
   Leu Ala Ala Val Glu Ile Arg Gly Val Ser Ser Pro Asp His Val Phe
       770                 775                 780
   Leu Pro Ile Pro Asn Trp Glu His Lys Glu Asn Pro Glu Thr Glu Glu
   785                 790                 795                 800
   Asp Val Gly Pro Val Val Gln His Ile Tyr Glu Leu Arg Asn Asn Gly
                   805                 810                 815
   Pro Ser Ser Phe Ser Lys Ala Met Leu His Leu Gln Trp Pro Tyr Lys
               820                 825                 830
   Tyr Asn Asn Asn Thr Leu Leu Tyr Ile Leu His Tyr Asp Ile Asp Gly
           835                 840                 845
   Pro Met Asn Cys Thr Ser Asp Met Glu Ile Asn Pro Leu Arg Ile Lys
       850                 855                 860
   Ile Ser Ser Leu Gln Thr Thr Glu Lys Asn Asp Thr Val Ala Gly Gln
   865                 870                 875                 880
   Gly Glu Arg Asp His Leu Ile Thr Lys Arg Asp Leu Ala Leu Ser Glu
                   885                 890                 895
   Gly Asp Ile His Thr Leu Gly Cys Gly Val Ala Gln Cys Leu Lys Ile
               900                 905                 910
   Val Cys Gln Val Gly Arg Leu Asp Arg Gly Lys Ser Ala Ile Leu Tyr
           915                 920                 925
   Val Lys Ser Leu Leu Trp Thr Glu Thr Phe Met Asn Lys Glu Asn Gln
       930                 935                 940
   Asn His Ser Tyr Ser Leu Lys Ser Ser Ala Ser Phe Asn Val Ile Glu
   945                 950                 955                 960
   Phe Pro Tyr Lys Asn Leu Pro Ile Glu Asp Ile Thr Asn Ser Thr Leu
                   965                 970                 975
   Val Thr Thr Asn Val Thr Trp Gly Ile Gln Pro Ala Pro Met Pro Val
               980                 985                 990
   Pro Val Trp Val Ile Ile Leu Ala  Val Leu Ala Gly Leu  Leu Leu Leu
           995                 1000                 1005
   Ala Val  Leu Val Phe Val Met  Tyr Arg Met Gly Phe  Phe Lys Arg
       1010                 1015                 1020
   Val Arg  Pro Pro Gln Glu Glu  Gln Glu Arg Glu Gln  Leu Gln Pro
       1025                 1030                 1035
   His Glu  Asn Gly Glu Gly Asn  Ser Glu Thr
       1040                 1045
<210> 2
   <211> 788
   <212> PRT
   <213> Homo sapiens
<400> 2